(19) 

**Europäisches Patentamt**

**European Patent Office**

**Office européen des brevets**

(11) **EP 4 092 131 A1**

(12) **EUROPEAN PATENT APPLICATION**
published in accordance with Art. 153(4) EPC

(43) Date of publication:
**23.11.2022 Bulletin 2022/47**

(21) Application number: **21741963.9**

(22) Date of filing: **14.01.2021**

(51) International Patent Classification (IPC):
*C12Q 1/68* (2018.01)          *A01K 61/00* (2017.01)
*C12N 15/09* (2006.01)         *C12Q 1/6869* (2018.01)

(52) Cooperative Patent Classification (CPC):
**A01K 61/00; C12N 15/09; C12Q 1/68; C12Q 1/686;
C12Q 1/6869; Y02A 40/81**

(86) International application number:
**PCT/JP2021/001076**

(87) International publication number:
**WO 2021/145384 (22.07.2021 Gazette 2021/29)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **14.01.2020 JP 2020003663**

(71) Applicant: **Kao Corporation
Chuo-ku
Tokyo 103-8210 (JP)**

(72) Inventors:
• **MIYATA, Kaede
Haga-gun, Tochigi 321-3497 (JP)**
• **HONDA, Hiroshi
Haga-gun, Tochigi 321-3497 (JP)**
• **YAMANE, Masayuki
Haga-gun, Tochigi 321-3497 (JP)**

(74) Representative: **Vossius & Partner
Patentanwälte Rechtsanwälte mbB
Siebertstraße 3
81675 München (DE)**

(54) **METHOD FOR SURVEYING ECOSYSTEM IN WATER ENVIRONMENT BY USING ENVIRONMENTAL RNA**

(57)    The present invention provides a simple and highly accurate method for an ecological survey with low environmental burden, more specifically, a method for analyzing biological species living in a water environment. The method assesses biological species living in the water environment quantitatively and further identify them exhaustively by analyzing RNA contained in the water environment.

**EP 4 092 131 A1**

**Description**

Field of the Invention

The present invention relates to a method for an ecological survey of a water environment using environmental RNA.

Background of the Invention

There is a growing global interest in conservation and sustainable use of fishery resources, such as fish and algae. Ecological surveys of water environments containing fishery resources play an important role in conservation and management of fishery resources. In ecological surveys of water environments, fish, algae, and arthropods have long been used as model organisms which serve as indicators of ecosystems. However, conventional methods for ecological surveys of water environments, such as an approach of catching individual organisms by fishing, trapping, or the like and methods which are dependent on observations of individual organisms including visual observation and photography, require specialized knowledge for determining species identities, involve cost and labor, and place burden on environment. In addition, these methods are not sufficiently exhaustive or reproducible.

In recent years, biomonitoring techniques are attracting attention as methods for ecological surveys. In particular, analysis of DNA contained in environmental samples such as water and soil (so-called environmental DNA [eDNA]) is expected to achieve a significant breakthrough in ecological surveys. In many cases, eDNA derived from a water environment, for example, is considered to be DNA released from individual aquatic organisms into a water environment (Patent Literature 1). There have been attempts of ecological surveys of water environments using eDNA metabarcoding analysis. For example, universal PCR primer sets for eDNA metabarcoding analysis which target fish mitochondrial DNA have been developed and reported to be useful for quantitative monitoring of fish (Non-patent Literatures 1 and 2). However, given that eDNA is very stable and exists in an environment for a long period, the possibility of false positive detection (false detection) cannot be ruled out in an ecological survey using eDNA. Not necessarily all species estimated by metabarcoding analysis may exist in the environment from which samples have been collected, particularly because rivers and bays can be contaminated with eDNA of fish or the like derived from domestic wastewater and fishing ports. In fact, it has been shown that eDNA derived from landed fish exists abundantly in the ocean near fishing ports and has a severe impact on eDNA analysis.

Reports have suggested that environmental RNA (eRNA) exists in an environment, as well as eDNA. RNA molecules may exist in higher copy number in a cell as compared with corresponding DNA, but are degraded easily in general and therefore appear more unlikely to remain in the environment than DNA. Therefore, while eRNA is expected to better reflect the current status of organisms in an environment than eDNA, it has been unclear whether eRNA can practically serve as a detection marker of organisms in the environment. Recently, attempts to detect organisms in a water environment using eRNA have been reported (Non-patent Literatures 3 to 6). These reports have shown that eRNA can be used to detect specific fish species as well as eDNA, and that ecological surveys by metabarcoding analysis can be conducted. However, the composition of detected species did not differ from that of species detected by eDNA, and usefulness of eRNA against false detection has not been reported.

(Patent Literature 1) JP-A-2017-99376

(Non-patent Literature 1) Miya et al., Royal Society Open Science, 2015, 2(7):150088, doi: 10.1098/rsos.150088
(Non-patent Literature 2) Ushio et al., Metabarcoding and Metagenomics, 2018, 2:1-15
(Non-patent Literature 3) Tsuri et al., Abstracts from the 66th Annual Meeting of the Ecological Society of Japan, February 20, 2019. <http://www.esj.ne.jp/meeting/abst/66/P1-479.html>
(Non-patent Literature 4) Nakamichi et al., Abstracts from the 66th Annual Meeting of the Ecological Society of Japan, February 20, 2019.
<http://www.esj.ne.jp/meeting/abst/66/P1-481.html> (Non-patent Literature 5) Jian et al., Abstracts from the 66th Annual Meeting of the Ecological Society of Japan, February 20, 2019. <http://www.esj.ne.jp/meeting/abst/66/P2-074.html>
(Non-patent Literature 6) Ijichi et al., Abstracts from First Meeting of The eDNA Society, Tokyo, September 18, 2018, p.28, P55

Summary of the Invention

The present invention provides a method for analyzing biological species living in a water environment, comprising analyzing RNA contained in the water environment quantitatively to assess the biological species living in the water environment quantitatively.

Brief Description of Drawings

[Figure 1] Figure 1 shows comparisons between eDNA and eRNA metabarcoding analyses and a field survey: a. fish (sum of data from two observation sites); b. fish (observed in Shin-Naka-hashi); c. algae; d. arthropods.
[Figure 2] Figure 2 shows relationships between the number of sequencing reads detected by the eDNA and eRNA metabarcoding analyses and the number of individual organisms detected by the field survey: a. fish eDNA; b. algae eDNA; c. fish eRNA; d. algae eRNA.
[Figure 3] Figure 3 shows comparisons of the number of sequencing reads between the eDNA analysis and the eRNA analysis: a. fish; b. algae; c. arthropods. Error bar = SE.
[Figure 4] Figure 4 shows assessments of risks for false positive in the eDNA and eRNA metabarcoding analyses: a. to c. data of fish: FW, freshwater fish; SW, seawater fish; BW, brackish water fish; Unknown, unidentified species. d. to f. data of arthropods: Aquatic, aquatic arthropods; Terrestrial, terrestrial arthropods. $*p < 0.05$, $**p < 0.001$.

Detailed Description of the Invention

In the present specification, the term "water environment" encompasses oceans, rivers, lakes, ponds, swamps, tidal flats, glaciers, aquafarms, and water tanks and banks/shores and bottoms thereof. Water environments are preferably oceans, rivers, lakes, ponds, swamps, tidal flats, and glacier, not breeding water, and banks/shores and bottoms thereof in a natural environment.
In the present specification, "environment DNA (eDNA)" and "environmental RNA (eRNA)" refer to DNA and RNA, respectively, contained in environmental samples such as water, mud, rock surface, and soil.
There is need for simple and highly accurate methods for ecological surveys with low environmental burden.
The present inventors demonstrated usefulness of an eRNA metabarcoding analysis by comparing the results of classical ecological surveys in rivers and the results of metabarcoding analyses using eRNA and eDNA in water environmental samples.
The present invention relates a method for an ecological survey of a water environment using environmental RNA. More specifically, the present invention provides a method for analyzing biological species living in a water environment. The method includes analyzing RNA contained in a water environment (eRNA) exhaustively and/or quantitatively.
According to the method of the present invention, false positives can be reduced, and ecosystems in water environments can be surveyed more accurately, compared with methods for ecological surveys using conventional biomonitoring techniques, for example, metabarcoding analysis using eDNA.
In the method of the present invention, performing a quantitative analysis of eRNA contained in a water environment enables quantification of biological species from which the eRNA is derived. Further, exhaustive analysis of eRNA also enables exhaustive identification of biological species living in the water environment. This enables exhaustive identification of biological species living in the water environment and/or quantitative assessment of the abundance thereof (for example, biomass or the number of individual organisms, or increases or decreases thereof). In the method of the present invention, eRNA alone may be a subject of measurement, but both eRNA and eDNA may be analyzed. Comparing both analysis results enables more accurate ecological surveys excluding false positives and analyses of contamination sources and contamination degrees in water environments.
eRNA and eDNA contained in water environments can be prepared according to usual methods. For example, water environmental samples are obtained by collecting water from surface layers and deep parts of oceans, rivers, lakes, ponds, swamps, aquafarms, water tanks, and the like or mud from bottoms thereof, and then RNA or DNA can be extracted from the water environmental samples. RNA and DNA can be extracted from water environmental samples according to usual methods. For example, after filtration of samples, RNA or DNA contained in a filtrate can be extracted by the phenol/chloroform method or the acid guanidinium thiocyanate-phenol-chloroform extraction (AGPC) method or using commercially available RNA or DNA extraction reagents. Extracted RNA and DNA may be immediately used for analysis, but may be stored according to usual methods (for example, cryopreserved at -80°C or -20°C) .
Extracted eRNA derived from a water environmental sample may be used as a form of RNA for various analyses, but RNA may be converted to DNA. Preferably, eRNA derived from the water environmental sample is converted to cDNA by reverse transcription and then used for various analyses. Common reverse transcriptase or reverse transcription reagents can be used for reverse transcription of RNA. Examples of commercially available reverse transcription reagents include PrimeScript (registered trade name) Reverse Transcriptase series (Takara Bio Inc.) and SuperScript (registered trade name) Reverse Transcriptase series (Thermo Scientific).

Various methods which can be used for exhaustive analysis of RNA can be applied to analysis of extracted eRNA. Biological species living in a water environment are identified by performing the exhaustive analysis to investigate biological species from which extracted eRNA is derived. Examples of methods for exhaustive analysis of RNA include next-generation RNA sequencing and RNA metabarcoding. Of these, RNA metabarcoding is preferred. RNA metabarcoding is a method in which a library is prepared by selectively amplifying cDNA derived from a marker RNA useful for determination of species, for example, 12S rRNA and 16S rRNA, among cDNA prepared from the sample RNA, the library is sequenced, and the determined sequences are checked against the genome sequence coding for the marker RNA of each biological species. A region useful for determination of species can be selectively amplified by PCR using universal primers constructed for fish, arthropods, algae, and the like, for example, MiFish (Non-patent Literature 1), which is a universal primer set for fish, gInsect (Bioengineering Lab. Co., Ltd.), which is a universal primer set for arthropods, or primers for photosynthetic organisms (psbA) (Bioengineering Lab. Co., Ltd.). Genome sequences of species to be checked against sequencing results can be obtained using a tool such as BLAST (<blast.ncbi.nlm.nih.gov/Blast.cgi>).

Quantitative analysis of extracted eRNA can be performed for some of biological species from which the eRNA identified by the above-described exhaustive analysis is derived, and exhaustive quantitative analysis, which is performed for the identified biological species exhaustively, is preferred. The amount of eRNA derived from each species identified by the above-described exhaustive analysis is examined by the quantitative analysis to quantify biological species living in a water environment. Quantitative analysis can be performed on the basis of, for example, the number of sequencing reads obtained by the above-described next-generation sequencing and sequencing in RNA metabarcoding. The large number of reads and diversity of reads reflect large amounts of the eRNA species existing in the sample, that is, biological species living in the water environment from which the eRNA is derived, as well as diversity of the biological species. Further, the number of individual organisms of the biological species can be estimated from the amount of eRNA by preliminarily calculating a correlation between the quantitative value (for example, the number of sequencing reads) of eRNA derived from a predetermined biological species and the number of individual organisms of the biological species observed by an actual field survey.

In the present invention, biological species living at a water sampling point are identified and/or quantified by the above-described procedure using eRNA contained in a water environment. Since eRNA is less stable than eDNA and remains in a less amount in the environment, analysis of eRNA enables more accurate identification and/or quantification of biological species living in the water environment as compared with analysis of eDNA. In contrast, eDNA analysis also detects biological species derived from DNA contained in contamination sources contaminated by external environments (for example, food waste in domestic wastewater and fish landed at fishing ports) in addition to biological species living at the water sampling point (see the examples described later). Neither a method for efficiently identifying false positives in eDNA analysis nor a method which can estimate contamination sources containing eDNA has been reported. In other words, eRNA is the only indicator that can be used to analyze biological species living at the water sampling point exclusively. As compared with analysis using eDNA, exhaustive identification of biological species by eRNA metabarcoding analysis is hardly affected by contamination by external environments (for example, contamination by DNA of fish and the like contained in food waste contained in human domestic wastewater and DNA of fish and the like derived from fishing ports), resulting in less false positives (see the examples described later). Therefore, more accurate ecological surveys (identification and quantification of living biological species, and the like) are enabled by using eRNA as a biological species marker.

The eRNA analysis according to the present invention may be performed in combination with an analysis of eDNA contained in the same water environment. When eDNA is also analyzed together with eRNA in the present invention, exhaustive analysis and quantification of the eDNA can be performed using techniques similar to the above-described techniques for exhaustive analysis and quantification of RNA, for example, next-generation sequencing and DNA metabarcoding. Of these, DNA metabarcoding is preferred. DNA metabarcoding can be performed by a procedure similar to RNA metabarcoding, except that DNA extracted from samples is used. Information useful for an ecological survey of a water environment can be obtained by comparing results from the eRNA analysis and the eDNA analysis. For example, among biological species identified by the eDNA analysis, those which are not identified by the eRNA analysis can be identified as biological species which do not exist at the water sampling point. Additionally, releases of DNA derived from old carcasses contaminating a water environment and a large amount of RNA from fresh carcasses and the like cause an imbalance between the eDNA amount and the eRNA amount in biological species which do not live at the water sampling point. Therefore, as shown in the examples described later, the ratio between the quantitative value of eRNA and the quantitative value of eDNA in predetermined biological species (the eNA ratio: the eNA ratio can be expressed as, for example, expression (1) [number of reads of eRNA or eDNA (smaller one)]/[number of reads of eRNA or eDNA (larger one)], but not limited to this expression) is preferred as an indicator to assess false positives. For example, given that the eNA ratio according to the expression (1) for biological species living in a subject water environment is higher than the eNA ratio for biological species not living in the water environment, whether the biological species is false positive by false detection can be assessed on the basis of the eNA ratio. False positive biological species can be detected and excluded from a group of identified biological species by performing this assessment for the group of identified biological species exhaustively. Additionally, as shown in the examples described later, marked reduction in the eNA ratio for terrestrial arthropods contaminating the water environment may be observed not only in the case of the eRNA amount less than the eDNA amount but also in the case of the eDNA amount less than the eRNA amount. Therefore, biological species living in the water environment can be identified and/or quantified more accurately by performing eDNA analysis and eRNA analysis in combination than by performing eRNA analysis alone. Therefore, performing eDNA analysis and eRNA analysis in combination can contribute to ecological surveys with less false positives or assessments of contamination of the water environment with biological wastes derived from external environments (for example, biological wastes contained in domestic wastewater and wastes from fishing ports), for example, analyses of contamination sources or contamination degrees in the water environment.

The present specification further discloses substances, manufacturing methods, intended uses, methods, and the like below as exemplary embodiments of the present invention. However, the scope of the present invention is not limited to these embodiments.

[1] A method for analyzing biological species living in a water environment, comprising
analyzing RNA contained in the water environment quantitatively to assess the biological species living in the water environment quantitatively.

[2] The method according to [1], preferably further comprising analyzing the RNA contained in the water environment exhaustively to identify the biological species living in the water environment exhaustively.

[3] The method according to [1] or [2], wherein

the biological species preferably include fish, algae, and arthropods, and
are more preferably fish.

[4] The method according to any one of [1] to [3], wherein the analysis of the RNA is preferably performed by metabarcoding of the RNA.

[5] The method according to [4], wherein the quantitative analysis of the RNA is preferably performed on the basis of the number of sequencing reads obtained by the metabarcoding.

[6] The method according to any one of [1] to [5], preferably further comprising analyzing DNA contained in the water environment exhaustively and/or quantitatively.

[7] The method according to [6], wherein the analysis of the DNA is preferably performed by metabarcoding of the DNA.

[8] The method according to [7], wherein the quantitative analysis of the DNA is preferably performed on the basis of the number of sequencing reads obtained by the metabarcoding.

[9] The method according to any one of [6] to [8], preferably comprising identifying the biological species living in the water environment exhaustively by comparing results of the analysis of the RNA and results of the analysis of the DNA.

[10] The method according to [9], wherein the exhaustive identification of the biological species preferably comprises identification of false positive biological species.

[11] The method according to any one of [6] to [10], preferably comprising assessing the biological species living in the water environment quantitatively by comparing results of the analysis of the RNA and results of the analysis of the DNA.

[12] The method according to [10], wherein the false positive biological species are preferably identified on the basis of a ratio between a quantitative value of RNA and a quantitative value of DNA for the biological species.

[13] The method according to [11], wherein the ratio between a quantitative value of the RNA and a quantitative value of the DNA for the biological species is preferably expressed as the following eNA ratio:
eNA ratio = [number of sequencing reads of the RNA or the DNA for the biological species (smaller one)]/[number of sequencing reads of the RNA or the DNA for the biological species (larger one)]

[14] The method according to any one of [1] to [13], wherein the water environment

preferably includes oceans, rivers, lakes, ponds, swamps, tidal flats, glaciers, aquafarms, and water tanks and banks/shores and bottoms thereof,

more preferably includes oceans, rivers, lakes, ponds, swamps, tidal flats, and glaciers, not breeding water, and banks/shores and bottoms thereof in a natural environment.

Examples

The present invention is explained more specifically below through examples. However, the scope of the present invention is not limited to these examples.

Example 1: Metabarcoding of eDNA and eRNA derived from water environmental samples

1) Preparation of eDNA and eRNA

The water environmental samples used in the study were collected over a period between July 2018 and February 2019 from two sites: Naka-gawa Ohashi (N36°32'55", E140°19'34") and Shin-Naka-hashi (N36°45'26", E140°08'30") located in the middle reaches of Naka-gawa (Naka River) in Japan. Using a bucket, 1.5 L of water was collected from the surface layer of the river. The water samples were immediately filtered using Sterivex™ Filter Unit (pore size, 0.45 $\mu$m: Millipore).
eDNA and eRNA were extracted from the water samples. DNeasy Blood and Tissue Kit (QIAGEN) was used to extract eDNA, and 15% polyvinylpolypyrrolidone (PVPP) solution was added to Buffer AL (containing RNaseA and Proteinase K) in the kit at a final concentration of 2.5%. The extracted eDNA was purified using MPure Bacterial DNA Extraction Kit (MP Biomedicals) and AMPure XP (Beckman Coulter). eRNA was extracted according to a recommended protocol using ChargeSwitch Total RNA Cell Kit (Thermo Fisher Scientific). cDNA was synthesized according to a recommended protocol using the eRNA as a template and PrimeScript II 1st strand cDNA Synthesis Kit (Takara Bio Inc.). Extraction of eDNA and eRNA and cDNA synthesis were performed in a similar manner using deionized water as a control, and extraction of eDNA and eRNA and occurrence of cross-contamination during cDNA synthesis were checked.

2) Preparation of amplicon library

An amplicon library of partial sequences of the 12S rRNA gene, the 16S rRNA gene, and the psbA gene was prepared using fish primers MiFish-U and MiFish-E33 (Non-patent Literature 1), gInsect, and psbA (Bioengineering Lab. Co., Ltd.). The amplicon library was prepared by a two-step tailed PCR method. In the first PCR, as a template, a solution obtained by diluting the eDNA solution prepared in 1) 3-fold with DDW was used for eDNA, and the cDNA solution prepared in 1) was used for cDNA. The concentration of each primer was set to be 0.5 $\mu$M. Using the MiFish-U, MiFish-E33, and gInsect primers, PCR was performed as: 95°C for 3 minutes, then 35 cycles of 98°C for 20 seconds, 65°C for 15 seconds, and 52°C for 30 seconds, followed by final elongation at 72°C for 5 minutes. Using the psbA primers, PCR was performed as: 94°C for 2 minutes, then 30 cycles of 94°C for 30 seconds, 52°C for 30 seconds, and 72°C for 30 seconds, followed by final elongation at 72°C for 5 minutes. The first PCR was performed for each eDNA and cDNA sample four times, and the products obtained from four PCRs were combined and used for the second PCR. A 0.5 $\mu$M primer pair containing a MiSeq adaptor sequence and an 8-bp index sequence was used in the second PCR, and the adaptor sequence and the index sequence were ligated to either end of the amplicon. The second PCR was performed as: 94°C for 2 minutes, then 10 to 12 cycles of 94°C for 30 seconds, 60°C for 30 seconds, and 72°C for 30 seconds, followed by the final elongation at 72°C for 5 minutes. The obtained amplification product was used as an amplicon library.

3) MiSeq sequencing, assembling, and BLAST search

Sequencing was performed by using the obtained amplicon library for Illumina MiSeq Reagent Kit v3 (600 Cycles) for 2 × 300-bp PE (Illumina). Using FASTX-Toolkit (<hannonlab.cshl.edu/fastx_toolkit/>: Hannon Laboratory, Cambridge University), 40 bp were removed from the primer sequence end of each read obtained by sequencing. Reads of QS20 or lower were removed. Assembling was performed to obtain a fragment length after assembling of 180 bp, a read fragment length of 170 bp, and the minimum overlap length of 10 bp. Reads which did not fulfil the above conditions were discarded. Nucleotide sequences which were considered identical, that is reads having 97% or higher identity, were assembled using USEARCH, and BLAST search was performed.

Example 2: Assessment of performance of metabarcoding in biota assessment for ecological survey

1) Field survey

A field survey (traditional field survey; TFS) of algae and arthropods was conducted. The TFS of arthropods was conducted according to the method described in "Census manual for river environment," 27th edition (River Environment Division, Water and Disaster Management Bureau, Ministry of Land, Infrastructure, Transport and Tourism) at the same survey site in the same season as for the water environmental samples. The samples were collected by qualitative sampling using a collecting net and quantification sampling with a 25 cm$^2$ × 25 cm$^2$ quadrat (each grid is a 250-$\mu$m. square). When the quadrat was used, sampling was performed at three sampling points in each survey site in each season. The TFS of algae focused on Lithophytic algae. Specifically, three stones each were collected from the same survey sites in the same season as for the water environmental samples, and algae were scraped from a 5 cm × 5 cm area of each stone. Since highly invasive surveys of fish are prohibited, except for ecological surveys initiated by the national government, this survey used the Naka-gawa data from the river environment database based on the census of riverbanks conducted by Ministry of Land, Infrastructure, Transport and Tourism.

2) Assessment of performance of eDNA and eRNA metabarcoding analyses

To assess validity of the eDNA and eRNA metabarcoding analyses, the number of species of fish, algae, and arthropods identified from eDNA or eRNA was compared with the number of biological species identified by the field survey (TFS). A group of living biological species determined by the TFS was identified as a true positive group, and the sensitivity and the positive predictivity for metabarcoding were calculated by the following equations:

$$\text{Sensitivity (\%) = \{(number of biological species belonging to the true positive group detected by eDNA or eRNA metabarcoding)/(total number of biological species belonging to the true positive group)\}} \times 100$$

$$\text{Positive predictivity (\%) = \{(number of biological species belonging to the true positive group detected by eDNA or eRNA metabarcoding)/(total number of biological species detected by eDNA or eRNA metabarcoding)\}} \times 100$$

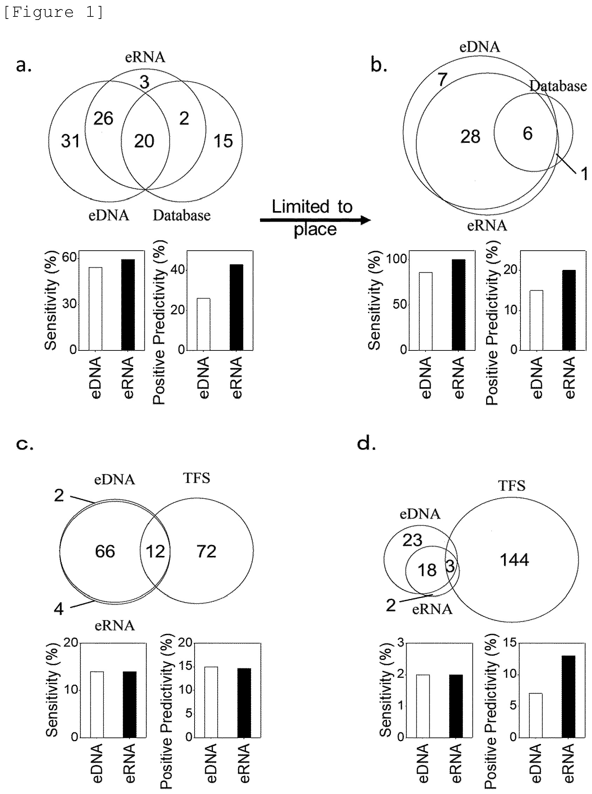

Figure 1 is Venn diagrams showing species of fish, algae, and arthropods detected by eDNA and eRNA metabarcoding analyses and a TFS, and graphs showing the sensitivity and the positive predictivity of the eDNA and eRNA analyses.

The eDNA and eRNA analyses detected 77 species and 51 species, respectively, of fish, which included 20 species and 22 species, respectively, among 37 species detected by the TFS. The eDNA and eRNA analyses detected 46 common species. The sensitivity (59%) of the eRNA analysis was slightly higher than the sensitivity (54%) of the eDNA analysis, and the positive predictivity (43%) of the eRNA analysis was much higher than the positive predictivity (26%) of the eDNA analysis (Figure 1a). When the observation site was limited to Shin-Naka-hashi (Figure 1b), the eRNA analysis detected all the fish species detected by the TFS, whereas the eDNA analysis missed one species. The sensitivity and the positive predictivity for detection of fish species at this observation site were 100% and 20%, respectively, in the eRNA analysis, indicating that, while false negatives were excluded, the results may have included false positives and true positives detected by the eRNA analysis but not detected by the TFS.

The eDNA and eRNA analyses detected 80 species and 82 species, respectively, of algae, which corresponded to the number of species detected by the TFS (84 species). However, only 12 species were detected commonly with the TFS. In contrast, almost all species detected by the eDNA and eRNA analyses (78 species) were common in these analyses, and the sensitivity and the positive predictivity of the eDNA and eRNA analyses were comparable (sensitivity, 14%; positive predictivity, 15%) (Figure 1c). One of causes for discrepancies between the results of the eDNA and eRNA analyses and the results of the TFS was considered that the TFS focused on algae attached to river stones, whereas eDNA and eRNA were collected from surface water.

The eDNA and eRNA analyses detected 44 species and 23 species, respectively, of arthropods, and the TFS detected 147 species. The eDNA and eRNA analysis detected 21 common species, but the eDNA and eRNA analyses and the TFS detected only three common species. The eDNA and eRNA analyses had comparable sensitivity of 2%.

The positive predictivity of the eRNA analysis (13%) was higher than that of the eDNA analysis (7%) (Figure 1d) .

The above results showed that eRNA, as well as eDNA, existed in a water environment in a sufficient amount for metabarcoding analysis. The detection sensitivity and the positive predictivity for algae and arthropods are lower than those for fish, but it may be attributed to unknown genome sequences contained in algae and arthropods. Genome sequences of algae and aquatic arthropods have been determined insufficiently as compared with those of fish, and cryptic species, which are hardly detected by morphological differences, may also exist. Further determination of genomes of algae and arthropods is awaited.

Example 3: Assessment of performance of metabarcoding in ecological surveys

1) Estimation of biomass

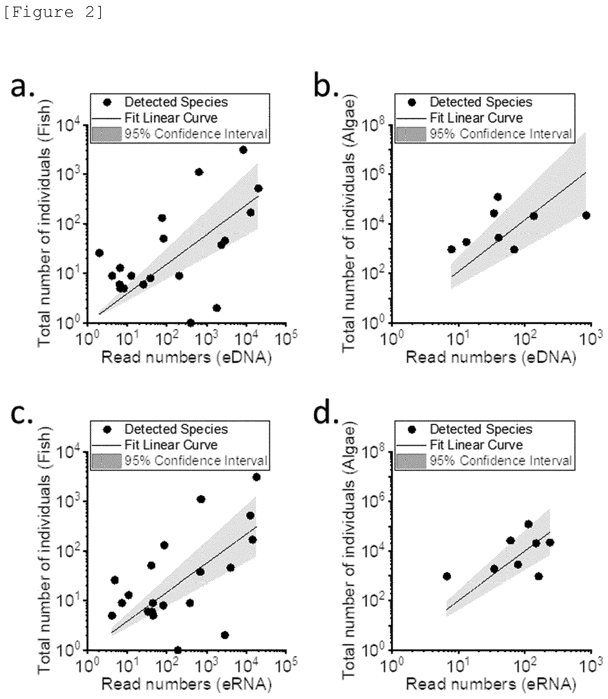

Characteristics of eDNA and eRNA analyses were assessed as quantitative biomass assessment methods. Figure 2 are scatter diagrams in which the number of individual organisms for each species (depending on the biomass) detected by the TFS is plotted against the number of sequencing reads calculated by the eDNA or eRNA metabarcoding analysis. Figures 2a and 2b show plots against the results of the eDNA analysis, and Figures 2c and 2d are plots against the results of the eRNA analysis. The mean value of total numbers of sequencing reads in the eDNA and eRNA metabarcoding analyses performed at two observation sites at the same timepoints were calculated and defined as the numbers of reads by the eDNA and eRNA analyses, respectively. Of note, species with a read length shorter than 10 bp in both eDNA and eRNA analyses were excluded from plotting, and 1 was added to all the numbers of reads to further draw on a logarithmic scale.

For fish and algae, all the numbers of sequencing reads in the eDNA and eRNA analyses correlated with the number of individual organisms (biomass) in the TFS (eDNA and eRNA of fish: $p = 2.95E^{-12}$ and $p = 1.53E^{-12}$, respectively, by F test; Spearman's correlation coefficients $\varepsilon = 0.49$ [p = 0.03] and f = 0.47 [p = 0.04], respectively. eDNA and eRNA of algae: $p = 2.91E^{-14}$ and $p = 2.08E^{-20}$, respectively, by F test; Spearman correlation coefficients f = 0.25 [p = 0.55] and f = 0.26 [p = 0.53], respectively). Correlation strengths of eDNA and eRNA were equal. Therefore, it was shown that both eRNA and eDNA could be used as an indicator to assess the biomass existing in a water environment for the fish and algae already detected by the TFS. In contrast, as described later, eDNA had a high false positive risk, and the quantitative value of eDNA was often calculated for biological species which did not live at the water sampling point. As a result, when a correlation between the number of sequencing reads and the number of individual organisms (biomass) was analyzed for biological species including those which were not detected by the TFS, eRNA had a stronger correlation than eDNA. Therefore, eRNA analysis was considered superior to eDNA analysis as a technique for quantitative assessment of biological species in an ecosystem in place of field surveys.

2) False positive risk assessment

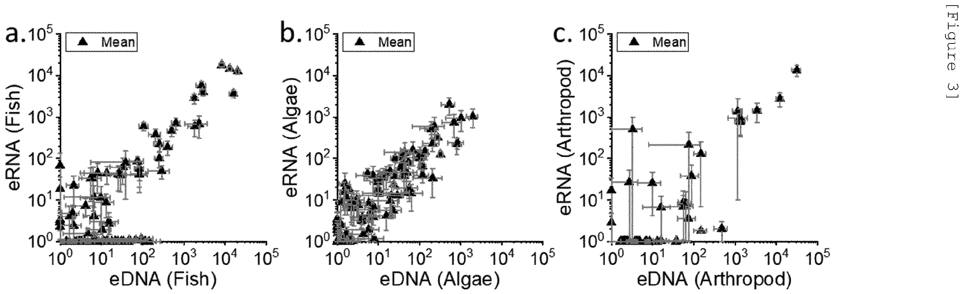

Figure 3 are scatter diagrams in which the numbers of sequencing reads in eDNA analysis were plotted against those of eRNA analysis for fish, algae, and arthropods. The number of reads of fish, algae, and arthropods in the eRNA analysis was similar to the number of reads in the eDNA analysis, and positive correlations were observed.

The false positive risks (e.g., detection rate) were assessed for the eDNA analysis and the eRNA analysis. Since the samples for the metabarcoding analyses were collected from the middle reaches of the river, detections of seawater fish and brackish water fish can be considered false positive, and detections of terrestrial arthropods may also be false positive. In this assessment, seawater and brackish water fish and terrestrial arthropods were considered as false positives, and false positive risks of the eDNA and eRNA analyses were examined. Further, the eNA ratio was obtained from the ratio of the numbers of reads of eRNA and eDNA by the following equation to assess their false positive risks.

$$\text{eNA ratio} = [\text{number of reads in eRNA or eDNA (smaller one)}]/[\text{number of reads in eRNA or eDNA (larger one)}]$$

Further, sensitivity and specificity of the eDNA analysis, the eRNA analysis, and the eNA ratio were obtained by the following equations to draw a Receiver Operatorating Characteristic (ROC) curve, and efficacy as an indicator which can exclude false positives was assessed.

$$\text{Sensitivity (\%)} = \{(\text{number of biological species}$$

$$\text{belonging to freshwater fish or aquatic arthropods}$$

$$\text{detected by eDNA or eRNA metabarcoding when the number of}$$

$$\text{reads as a threshold was gradually changed)/(number of}$$

$$\text{biological species belonging to freshwater fish or}$$

$$\text{aquatic arthropods detected by eDNA or eRNA}$$

$$\text{metabarcoding)} \} \times 100$$

$$\text{Specificity (\%)} = \{(\text{number of biological species}$$

$$\text{belonging to seawater and brackish water fish or}$$

$$\text{terrestrial arthropods detected by eDNA or eRNA}$$

$$\text{metabarcoding when the number of reads as a threshold was}$$

$$\text{gradually changed)/(number of biological species}$$

$$\text{belonging to seawater and brackish water fish or}$$

$$\text{terrestrial arthropods detected by eDNA or eRNA}$$

$$\text{metabarcoding)} \} \times 100$$

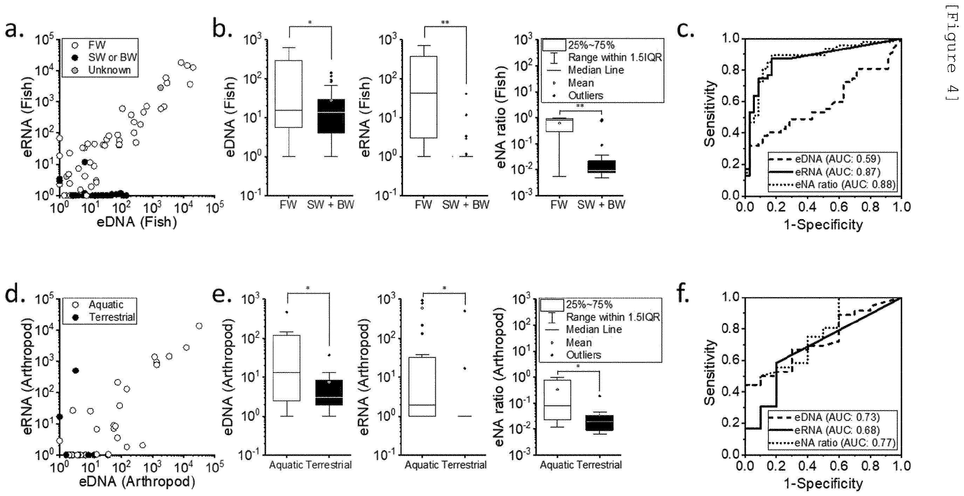

Figure 4a shows a scatter diagram in which the numbers of sequencing reads in the eDNA analysis were plotted against those in the eRNA analysis for freshwater fish (FW), seawater fish (SW), brackish water fish (BW), and unidentified fish species (Unknown). Figure 4b shows box-and-whisker plots of the number of reads in eDNA, the number of reads in eRNA, and the eNA ratio for freshwater fish (FW) and seawater fish (SW) plus brackish water fish (BW). Figure 4c shows ROC curves. Twenty nine of 36 species which were identified in the eDNA analysis but almost none of which reads were detected in the eRNA analysis were seawater fish (SW) or brackish water fish (BW) which are considered not to exist naturally in the water environment (middle reach of river) from which samples were collected. These results showed that the eDNA analysis detected biological species not existing in a water environment as false positives, and therefore was not suitable as a method for accurately assessing biological species living in the water environment. Additionally, the number of eRNA reads and the eNA ratio of freshwater fish (FW) living in the water environment from which samples were collected showed a statistically significantly higher and clearly different distribution than the eNA ratio of seawater and brackish water fish (SW + BW) not living in the water environment from which samples were collected (Figure 4b: *p < 0.05, **p < 0.001; Spearman's correlation test and Wilcoxon's signed-rank sum test were performed because F test showed unequal variance). While the ROC analysis showed that the eRNA and the eNA ratio had high efficacy as an indicator that could exclude false positives, eDNA showed no efficacy in exclusion of false positives (eRNA, AUC = 0.88; eNA ratio, AUC = 0.87; eDNA, AUC = 0.59: Figures 4b and 4c).

Figures 4d to 4f show the results of analyses for aquatic arthropods (Aquatic) and terrestrial arthropods (Terrestrial) which were similar to the analyses in Figures 4a to 4c. The eRNA and the eNA ratio of arthropods were shown to have high efficacy as an indicator which could exclude of false positives, that is, false detection of terrestrial arthropods (eRNA, AUC = 0.68; eNA ratio, AUC = 0.77: Figures 4e and 4f).

The significant reduction in the eNA ratio of seawater and brackish water fish is primarily attributed to the eRNA amount less than the eDNA amount. This finding was considered to be because eDNA of organisms not living in a water environment is accumulated in the water environment, but eRNA of these organisms is degraded and not accumulated in the water environment, resulting in the difference in the abundance ratio of eDNA and eRNA. Additionally, only eDNA may remain, and eRNA may have already been degraded in domestic wastewater containing nucleic acids of fish that may contaminate river water. In contrast, the significant reduction in the eNA ratio of terrestrial arthropods was observed with two cases of the eRNA amount less than the eDNA amount and the eDNA amount less than the eRNA amount. The former case may occur because nucleic acids including more eDNA may be dissolved from individual organisms which have died and whose eRNA degradation has been advanced near the river into river water, and the latter case may occur because nucleic acids of individual organisms who have died a short time earlier and in which RNA is more abundant than DNA flow out to the river water, and then the eRNA concentration is maintained in a less flowable environment. In either case, since change in the ratio of the eDNA amount and the eRNA amount observed in the contamination sources from external environments can be observed as a significant reduction in the eNA ratio, biological species with a markedly low eNA ratio may not be biological species living in the water environment. Additionally, because, as described earlier, certain quantitative values for species which are false positive are calculated in the eDNA analysis, assessment methods using eRNA or eDNA and eRNA in combination were considered to be effective in quantitative assessment. The above results showed that eRNA and eNA ratio were effective as indicators to exhaustively identify or quantify biological species of fish and arthropods living in a water environment without false detection of species not living in the water environment.

**Claims**

1. A method for analyzing biological species living in a water environment, comprising
analyzing RNA contained in the water environment quantitatively to assess the biological species living in the water environment quantitatively.

2. The method according to Claim 1, further comprising analyzing the RNA contained in the water environment exhaustively to identify the biological species living in the water environment exhaustively.

3. The method according to Claim 1 or 2, wherein the biological species include fish, algae, and arthropods.

4. The method according to Claim 3, wherein the biological species are fish.

5. The method according to any one of Claims 1 to 4, wherein the analysis of the RNA is performed by metabarcoding of the RNA.

6. The method according to any one of Claims 1 to 5, further comprising analyzing DNA contained in the water environment exhaustively and/or quantitatively.

7. The method according to Claim 6, comprising identifying the biological species living in the water environment exhaustively by comparing results of the analysis of the RNA and results of the analysis of the DNA.

8. The method according to Claim 7, wherein the exhaustive identification of the biological species includes identification of false positive biological species.

[Figure 1]

[Figure 2]

[Figure 3]

[Figure 4]

**INTERNATIONAL SEARCH REPORT**

International application No.

PCT/JP2021/001076

### A. CLASSIFICATION OF SUBJECT MATTER

C12Q 1/68(2018.01)i; A01K 61/00(2017.01)i; C12N 15/09(2006.01)i; C12Q 1/6869(2018.01)i
FI: C12Q1/68; A01K61/00; C12Q1/6869 Z; C12N15/09 Z

According to International Patent Classification (IPC) or to both national classification and IPC

### B. FIELDS SEARCHED

Minimum documentation searched (classification system followed by classification symbols)
C12Q1/68; A01K61/00; C12N15/09; C12Q1/6869

Documentation searched other than minimum documentation to the extent that such documents are included in the fields searched

| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)
JSTPlus/JMEDPlus/JST7580 (JDreamIII); CAplus/MEDLINE/EMBASE/BIOSIS/WPIDS (STN)

### C. DOCUMENTS CONSIDERED TO BE RELEVANT

| Category* | Citation of document, with indication, where appropriate, of the relevant passages | Relevant to claim No. |
|---|---|---|
| X | 中道友規 ほか，環境 RNA と環境 DNA による魚類の検出感度の比較とその時間変化，日本生態学会第 66 回全国大会講演要旨，一般講演 ポスター発表, 2019, P1-481, entire text, (NAKAMICHI, Tomoki et al., "Comparison of the sensitivities of environmental DNA and RNA as markers for species detection from water samples and their time dependencies", Lecture abstracts of the 66th Annual Meeting of the Ecological Society of Japan, General lecture poster presentation) | 1-8 |

☒ Further documents are listed in the continuation of Box C.  ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 16 March 2021 (16.03.2021) | 30 March 2021 (30.03.2021) |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

**INTERNATIONAL SEARCH REPORT**

International application No.

PCT/JP2021/001076

C (Continuation). DOCUMENTS CONSIDERED TO BE RELEVANT

| Category* | Citation of document, with indication, where appropriate, of the relevant passages | Relevant to claim No. |
|---|---|---|
| A | 姜明揚 ほか，野外におけるコイの環境 RNA の検出，　日本生態学会第 66 回全国大会講演要旨，一般講演 ポスター発表，2019, P2-074, entire text, non-official translation (JIANG, Ming-Yang et al., "Detection of environmental RNA of cyprinus carpio in the field", Lecture abstracts of the 66th Annual Meeting of the Ecological Society of Japan, General lecture poster presentation) | 1-8 |
| A | USHIO, M. et al., "Quantitative monitoring of multispecies fish environmental DNA using high-throughput sequencing", Metabarcoding and Metagenomics, 2018, 2: e23297, abstract | 1-8 |
| A | MIYA, M. et al., "MiFish, a set of universal PCR primers for metabarcoding environmental DNA from fishes: detection of more than 230 subtropical marine species", R. Soc. Open Sci., 2015, 2: 150088, abstract | 1-8 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **MIYA et al.** *Royal Society Open Science,* 2015, vol. 2 (7), 150088
- **USHIO et al.** *Metabarcoding and Metagenomics,* 2018, vol. 2, 1-15
- **TSURI et al.** *Abstracts from the 66th Annual Meeting of the Ecological Society of Japan,* 20 February 2019, http://www.esj.ne.jp/meeting/abst/66/P1-479.html
- **NAKAMICHI et al.** *Abstracts from the 66th Annual Meeting of the Ecological Society of Japan,* 20 February 2019, http://www.esj.ne.jp/meeting/abst/66/P1-481.htm
- **JIAN et al.** *Abstracts from the 66th Annual Meeting of the Ecological Society of Japan,* 20 February 2019, http://www.esj.ne.jp/meeting/abst/66/P2-074.html
- **IJICHI et al.** *Abstracts from First Meeting of The eDNA Society, Tokyo,* 18 September 2018, 28, , 55